Europäisches Patentamt

(19) European Patent Office

Office européen des brevets

(11) Publication number: **0 152 746 B1**

(12) **EUROPEAN PATENT SPECIFICATION**

(45) Date of publication of patent specification: **14.08.91**    �select (51) Int. Cl.⁵: **C12N 15/12, C12P 21/00**

(21) Application number: **85100224.6**

(22) Date of filing: **11.01.85**

Divisional application 91100360.6 filed on 11/01/85.

(54) Hybridoma cell lines and monoclonal antibodies to factor VIIIC.

(30) Priority: **26.10.84 US 664919**
**12.01.84 US 570062**
**07.01.85 US 689274**

(43) Date of publication of application:
**28.08.85 Bulletin 85/35**

(45) Publication of the grant of the patent:
**14.08.91 Bulletin 91/33**

(84) Designated Contracting States:
**AT BE CH DE FR GB IT LI LU NL SE**

(56) References cited:
**EP-A- 0 083 483**
**EP-A- 0 123 945**
**EP-A- 0 182 372**

**BIOLOGICAL ABSTRACTS, vol. 77, no. 6,
March 1984, page 4443, abstract no. 40713,
Biological Abstracts Inc., Philadelphia, US; F.
ROTBLAT et al.: "Monoclonal antibodies to
human procoagulant factor VIII"**

(73) Proprietor: **CHIRON CORPORATION**
**4560 Horton Street**
**Emeryville California 94608(US)**

Proprietor: **NOVO NORDISK A/S**
**Novo Allé**
**DK-2880 Bagsvaerd(DK)**

(72) Inventor: **Kuo, George**
**1370 6th Avenue**
**San Francisco California 94122(US)**
Inventor: **Masiarz, Frank R.**
**148 Marview Way**
**San Francisco, California 94131(US)**
Inventor: **Truett, Martha**
**9082 Broadway Terrace**
**Oakland, California 94611(US)**
Inventor: **Valenzuela, Pablo**
**455 Upper Terrace**
**San Francisco, California 94117(US)**
Inventor: **Rasmussen, Mirella Ezban**
**Abildgaardsgade 24**
**DK-2100 Copenhagen(DK)**
Inventor: **Favaloro, Jennifer**
**108 Faraday Street**
**Carlton 3035, Victoria(AU)**

CHEMICAL ABSTRACTS, vol. 98, no. 21, 23rd May 1983, page 450, abstract no. 176896j, Columbus, Ohio, US; C.A. FULCHER et al: "Thrombin proteolysis of purified factor VIII procoagulant protein: correlation of activation with generation of a specific polypeptide"

CHEMICAL ABSTRACTS, vol. 96, no. 5, 1st February 1982, page 480, abstract no. 33095v, Columbus, Ohio, US; H.P. MULLER et al.: "A monoclonal antibody to VIII:C produced by a mouse hybridoma"

PROCEEDINGS OF THE NATIONAL ACADEMY OF SCIENCES OF USA, vol. 79, March 1982, pages 1648-1652, Washington, US; C.A. FULCHER et al.: "Characterization of the human factor VIII procoagulant protein with a heterologous precipitating antibody"

BIOCHEMISTRY, vol. 24, 30th July 1985, pages 4294-4300, American Chemical Society, Easton, PA, US; F. ROTBLAT et al.: "Purification of human factor VIII:C and its characterization by Western Blotting using monoclonal antibodies"

(74) Representative: **Patentanwälte Beetz sen. - Beetz jun. Timpe - Siegfried - Schmitt-Fumian- Mayr Steinsdorfstrasse 10 W-8000 München 22(DE)**

**Description**

Factor VIIIC is a plasma protein that participates in the intrinsic pathway of blood coagulation. It is absent or defective in individuals with the hereditary X chromosome-linked recessive bleeding disorder hemophilia A. Great difficulty has been encountered in isolating Factor VIIIC due to its extremely low concentration in plasma and the fact that it appears to be an intermediate or final degradation product of a larger protein precursor. Efforts to isolate Factor VIIIC have led to complex mixtures of various polypeptides having significant heterogeneity and varying molecular weights.

It would be of great interest therefore to prepare monoclonal antibodies which are capable of selectively reacting with individual polypeptides or groups of polypeptides which are derived from Factor VIIIC.

US-A-4 361 509 and references cited therein describe purification of Factor VIIIC (see also Fulcher and Zimmerman, Proc. Natl. Acad. Sci. USA (1982) 79:1648-1652). Tuddenham et al., J. of Lab. Clinical Medicine (1979) 93:40-53 describe purification of Factor VIIIC using polyclonal antibodies. Austen, British J. Hematology (1979) 43:669-674 describes the use of aminohexyl-Sepharose for Factor VIIIC purification. Weinstein et al., Proc. Natl. Acad. Sci. USA (1981) 78:5137-5141 describe a study of the effect of thrombin on Factor VIII (see also Kuo et al., Abstracts for IX International Congress of Thrombosis and Hemostasis, Stockholm, July, 1983).

Hybritech Data Sheet (Hybritech, Inc., 11085 Torreyana Road, San Diego, California 92121) describes a monoclonal antibody (Catalog #0432) which is specific for human Factor VIIIC. DK-A-53519 describes the preparation of monoclonal antibodies to human factor VIIIC and polypeptide fragments thereof. Certain of the monoclonal antibodies described in the Danish patent application are also described by Zimmerman et al., Blood (1983) 61:807. Fass et al., Blood (1982) 59:594, report that active human Factor VIIIC may be eluted from monoclonal antibodies by ethylene glycol (50 %).

EP-A-123 945 discloses monoclonal antibodies of class IgG produced by a hybridoma cell line which react with human Factor VIIIC polypeptide fragments. None of these antibodies (cf. EP-A-123 945, pages 4 and 5) has such a specifity pattern as to react exclusively with the 80/77 kD doublet, and eventually with the 240 kD polypeptide fragment of Factor VIIIC.

It is the object of the present invention to provide particular hybridoma cell lines producing monoclonal antibodies having a specific reactivity pattern with Factor VIIIC fragments, and specifically reacting with the 80/77 kD doublet, and to provide the respective monoclonal antibodies.

The above object is achieved according to the claims.

The invention provides hybridoma cell lines 1-28, 47, 42 and 56, deposited with the National Collection of Animal Cell Cultures, Porton Down, Salisbury, Wilts, GB, under accession numbers 84122101, 84122102, 84122103 and 84122104, respectively, and the cell line 2B6 deposited with the American Type Culture Collection, 12301 Parklawn Drive, Rockville, Maryland 20852, US, under accession number HB 8690, and the monoclonal antibodies obtained from these hybridoma cell lines.

The invention further provides hybridoma cell lines which produce monoclonal antibodies having a reactivity substantially identical to that of the monoclonal antibodies obtained from the above-mentioned deposited hybridoma cell lines, and also the monoclonal antibodies obtained from these hybridoma cell lines.

Human Factor VIIIC is a complex protein which can be isolated in substantially pure form and exhibits an apparent molecular weight of about 460 kD. Upon electrophoresis under denaturing conditions, a large number of fragments of varying molecular weights result: 240, 160, 140, 115, 92.5, 80 and 77 kD, the latter two being found to migrate as a doublet. Analysis of the fragments by chemical and protease cleavage including thrombin, employing the monoclonal antibodies including those of the present invention to follow immunogenic relationships and cleavage patterns to follow structural relationships, demonstrates that the 92.5 kD polypeptide is related to the 240, 160, 140 and 115 polypeptides while the 77/80 kD doublet appears to have common antigenic characteristics with only the 240 kD polypeptide. It is further found that the 77/80 kD doublet is converted by thrombin to a 67/70 kD doublet, while the 92.5 kD polypeptide present in purified Factor VIIIC material treated with thrombin is cleaved by thrombin, directly or indirectly, into two polypeptides of about 40 and 52.5 kD.

The class I hybridomas of this invention produce monoclonal antibodies reactive with a 80/77 kD doublet fragment of Factor VIIIC or with both a 240 kD fragment and a 80/77 kD doublet

Monoclonal antibodies according to the present invention produced by cell lines 42, 47 and 56 are reactive with the 80/77 kD doublet. Monoclonal antibodies produced by cell line 2B6 react with the 80/77 kD doublet and with a 240 kD fragment.

The monoclonal antibodies of the present invention are useful for a variety of purposes. For example, the antibodies may be used to isolate the entire Factor VIIIC complex from plasma, as well as isolating the various polypeptide fragments of Factor VIIIC from a purified source. In addition, the antibodies may be used for detecting the presence of Factor VIIIC and related polypeptides in plasma by a variety of immunoassay techniques. Suitable immunoassay techniques are well known and amply described in the scientific and patent literature.

The monoclonal antibodies of the present invention may also be used to facilitate preparation of additional hybridomas producing monoclonal antibodies having substantially identical reactivity, but often differing in other characteristics, such as isotype, binding affinity, and the like. This can be achieved by using the monoclonal antibody to enrich these polypeptides carrying determinant sites recognized by the antibody in a source Factor VIIIC. Conveniently, an affinity column may be prepared using the desired antibody. By then applying the Factor VIIIC to the column in an appropriate buffer, polypeptides recognized by the antibody will be bound. After washing the column to assure removal of non-specifically bound proteins, the polypeptide of interest may be released from the column by adjusting the pH, employing urea, or by utilizing other mildly denaturing conditions. The antigen may then be isolated and used to hyperimmunize an appropriate host, and hybridomas produced by the now classical teachings of Kohler and Milstein (1975) Nature 256:495, or more recent adaptations thereof. Details of suitable immunization, immortalization, and screening procedures are set forth below. Following such procedures, a variety of antibodies having substantially identical reactivities, but differing in class, binding constant, host, and the like, can be obtained.

In general, hybridomas may be produced by first hyperimmunizing a mouse or other small mammal with the Factor VIII polypeptide of interest. Specific methods of immunization are well known and are amply described in the literature. The Factor VIII polypeptide is injected with or without adjuvants into the mammal followed by from 2 to 6 subsequent booster injections over a relatively short period of time. The animals are then killed, usually several days after the final injection, the spleens removed, and the spleen cells immortalized.

The manner of immortalization is not critical. Presently, the most common way is fusion with a myeloma cell fusion partner, which method is exemplified here. Other techniques include EBV transformation, transformation with bare DNA, e.g., oncogenes, retroviruses, etc., or any other method which provides for stable maintenance of the cell line and production of monoclonal antibodies.

When employing fusion with fusion partner, the manner of fusion is not critical, and various techniques may be employed. Conveniently, the spleen cells and myeloma cells are combined in the presence of polyethylene glycol, a suitable growth medium, and other additives for a few minutes. At the end of the fusion, the non-ionic detergent is removed by washing the cells.

The fused cells are then promptly dispensed into small culture wells, usually in a microtiter plate, at relatively low density for screening. The growth medium in the culture wells is selected to support growth of the hybrid cells while being lethal to the myeloma cells. Conveniently, the myeloma cell line has been mutated to be sensitive to particular agents, typically being HAT sensitive.

After sufficient time, usually from one to two weeks, colonies of hybrids are observed, and plates containing positive wells are identified. The plates and wells having only one colony per well are selected, and the supernatants from these wells are tested for binding activity against the particular polypeptide fragment(s) of Factor VIIIC. Other characteristics of the hybridoma cell line and monoclonal antibodies, such as growth characteristics of the hybridoma, binding affinity of the monoclonal antibodies, antibody class, cross-reactivity of the antibodies, and the like, can then be determined. Those hybridomas which produce antibodies having the desired specificity as well as other desirable characteristics can then be maintained as viable cultures and/or frozen storage.

The following examples are offered by way of illustration.

1. Purification of Factor VIIIC

Human Factor VIIIC was isolated from commercial cryoprecipitate preparations by a) immunosorbent chromatography using a polyclonal anti VIIIR-Sepharose column by a method first described by E.G.D. Tuddenham, N.C. Trabold, J.A. Collins, and L.W. Hoyer, J. of Lab. Clinical Medicine (1979) 93:40; and b) a chromatographic separation on aminohexyl-substituted agarose as was originally described by D.E.G. Austen, British J. of Hematology (1979) 43:669. Details of the procedures are described below.

Goat anti-human Factor VIII Related Antigen (VIIIR) serum obtained from Atlantic Antibody (cat. no. 040-01), was treated with either a standard 0-50 % ammonium sulfate cut followed by DEAE cellulose column chromatography, or a similar 0-33 % cut without subsequent chromatography. These materials were then conjugated to CNBr-activated Sepharose CL2B or 4B, respectively (Pharmacia, 17-0140-01 or 17-0430-01), and poured as a column (anti VIIIR-Sepharose column).

"HEMOFIL", a stable, dried preparation of antihemophilic factor (Factor VIII, AHF, AHG) in concentrated form prepared from fresh, normal human plasma, representing about a 40-fold enrichment for Factor VIIIC, was dissolved in the following buffer: 0.02 M imidazole, 0.15 M NaCl, 0.1 M lysine-HCl, 0.02 % NaN$_3$, pH 7.4.

After being dissolved, the Hemofil was applied to the above-described anti VIIIR-Sepharose column. Non-specifically bound protein was eluted with the above buffer modified to 0.5M NaCl. Next, Factor VIIIC was eluted with the above buffer containing 0.35 M CaCl$_2$, with the addition of 10 % glycerol which stabilizes the Factor VIIIC activity. Active fractions from the immunosorbent column were pooled and dialyzed against buffer (0.02 M imidazole, 0.15 M NaCl, 0.1 M lysine-HCl, 0.025 M CaCl$_2$, 0.02 % NaN$_3$, 10 % glycerol, pH 7.4). An aliquot of the dialyzed fractions, which contained 1,100 units of Factor VIIIC, was applied to an aminohexyl-Sepharose 4B column (1x6cm) equilibrated with dialysis buffer described above. Factor VIIIC activity was eluted with the same buffer containing either 0.35 M CaCl$_2$ or 2 M NaCl. The activity was found to be in a volume of 2 ml with 500 units of Factor VIIIC per ml. Subsequent experiments carried out in the same manner provided a recovery of 25 % off the anti VIIIR column and a recovery of approximately 90 % off the aminohexyl column. Alternatively, pooled, dialyzed material eluted from the immunosorbent column is first applied to a dextran sulfate agarose (Pharmacia) column (1.5 x 6cm) equilibrated with the dialysis buffer above and eluted with the same buffer. Several minor contaminants, e.g., fibrinogen, fibronectin, IgG, are retained on the column while Factor VIIIC emerges in the flow-through which is collected and loaded on the aminohexyl-Sepharose column as before.

Both biological, i.e., clotting, and antigenic (CAg) activity were shown to be present in the purified Factor VIIIC, as demonstrated by the subsequent assays indicating a 5,000-fold purification over the 40-fold concentration in Hemofil. Using a standard commercially available three component kit from General Diagnostics, (APTT, Factor VIII deficient plasma, Verify Normal Citrate) a coagulation assay was carried out and indicated high levels of Factor VIIIC biological activity.

### 2. Preparation of Monoclonal Antibodies

Balb/c mice were immunized with purified human Factor VIIIC, as described above. Spleen cells ($10^8$) were fused with $10^7$ NSO or $10^7$ NSI mouse myeloma cells (according to Kohler and Milstein (1975) Nature 256:495). The products of each fusion were plated onto two 96 well microtiter plates. A spleen cell feeder layer was used at $10^4$ cells/well. Colonies were visible microscopically from the fifth day, and the supernatants were assayed every few days for presence of antibodies against Factor VIIIC. An enzyme linked immunoadsorbent assay (ELISA) was used employing the following layers in an immunoassay plate (Nunc F2):

1st layer: Monospecific anti-mouse Ig
2nd layer: Hybridoma cell supernatant
3rd layer: Cryo-precipitate containing human Factor VIIIC
4th layer: Peroxidase labelled IgG from a hemophilic inhibitor patient.

Four positive cell lines were identified, purified by subcloning and grown in liter scale cultures in the laboratory. Three of the positive cell lines, designated 42, 47 and 56, were derived from fusions with the NSO myeloma cell line. The fourth positive cell line was derived from fusion with the NSI myeloma cell line and designated 1-28.

Other fusions were performed in the manner just described except that the fusion partner was cell line P3X63Ag8,653 (Kearney et al. (1979) J. Immunol. 123:1548-1550). ELISA screening was performed with the following layers:

1st layer: Purified Factor VIIIC
2nd layer: Hybridoma cell supernatant
3rd layer: Horseradish peroxidase (HRP)-labelled goat anti-mouse IgG
4th layer: HRP-substrate

Five positive cell lines were identified, subcloned, grown in liter scale cultures and characterized as described in Section 4. The cell lines were designated as 1B9, 2B6, 2F9, 4E5 and 5B1.

### 3. Amplification of Positive Hybridomas

In order to be certain that each hybridoma producing anti-Factor VIII antibodies was free from cells producing other immunoglobulins and cells not producing immunoglobulins, purification by subcloning was performed. Dilutions of hybridoma cells 42, 47, 56 and 1-28 were seeded in microtiter plates, such that each well theoretically contained a single cell. When necessary because of low cloning efficiency, initial seeding was at two cells per well followed by a second seeding at one cell per well. After 7 to 14 days, supernatants from each colony of presumptively identical cells derived from each hybridoma cell line were screened to identify the anti-Factor VIII producing colonies. The positive subclones were then expanded to macroculture.

Multiple aliquots of each cell line were frozen in liquid nitrogen as soon as purification was complete. This provided amplification of the cultures from the original single microwell ($10^2$ cells in 0.2 ml) to about $10^8$ cells, which corresponded to about 100 ml of medium. The cells were very sensitive during this stage of growth, and improper handling could result in death of the culture. Although individual characteristics of each cell line meant that identical procedures could not be used for all four lines, the following steps were generally employed to keep the cultures alive and growing:

1. Micro wells were approximately $\frac{1}{4}$ confluent before transfer to macroculture.

2. Pre-conditioned medium was used for the first two weeks of subculture.

3. Transfer from macro to flask culture was delayed until at least 4 ml of macroculture was grown to half-confluence.

4. Dilution of the flask culture was limited to less than 1:2 for the first few passages.

4. Characterization of Monoclonal Antibodies

To identify the immunoglobulin class of the monoclonal anti-Factor VIIIC antibodies, an immunoassay for mouse IgG was carried out in which the coating antibodies were specific for immunoglobulin-subclasses. Microplates precoated with anti-isotype antibodies were obtained from Hagedorn Research Lab. The antibodies produced by hybridoma cell lines 42, 47 and 56 were found to be IgG 1.

The class of the monoclonal antibodies was identified according to their reactivity with Factor VIIIC polypeptides using the following procedure. Hybridoma culture supernatants were incubated for 12 hours at room temperature with polystyrene beads (1/8" diameter, Precision Plastic Ball Co., Chicago) coated with affinity-purified goat anti-mouse immunoglobulin (IgG). The polystyrene beads were washed with phosphate-buffered saline and reacted with purified Factor VIIIC labelled with $^{125}$I using the chloramine T method. The labelled proteins bound on the beads were solubilized in SDS sample buffer for gel electrophoresis and were separated on an 7.5 % acrylamide gel (Laemmli Nature (1970) 227:280). The gel was fixed, dried and exposed to Kodak X-Omat film with an intensifying screen for 20 hours or longer at -80 °C. The results obtained with this procedure allowed classification of the monoclonal antibodies according to the invention as being of Class I i.e. react with the 80/77 kD doublet or with the 80/77 kD doublet and the 240 kD protein fragment. These antibodies also react with a 70/67 kD doublet when thrombin-digested Factor VIII is used in the assay, indicating that the 70/67 kD doublet produced by thrombin digestion, is derived from the 80/77 kD doublet (monoclonal antibodies obtained from cell lines 2B6, 42, 47, 56, 1-28).

The antibodies were screened for Factor VIIIC coagulation inhibition activity using the conventional Bethesda assay (C. Kasper (1975) Thrombos. Diathes. Haemorrh. 34:869-782). Of the above antibodies, only those produced by cell line 56 displayed coagulation inhibition activity.

The following experiments were performed to study the association between the 92.5 kD protein and the 80/77 kD doublet, as well as to investigate the role of calcium in the association. The following ELISA assay were performed in the presence of 10 mM CaCl$_2$ or in the presence of the same concentration of CaCl$_2$, 10 mM EGTA, and 10 mM EDTA.

1st layer: Monospecific anti(mouse IgG)

2nd layer: Class III monoclonal antibody (anti-92.5 kD) or Class I monoclonal antibody (anti-80/77 kD)

3rd layer: Purified Factor VIIIC material

4th layer: HRP-human inhibitor antibody to 80/77 kD.

The results are shown in the Table. As the table shows, a strong HRP reaction is obtained when only calcium is present in the assay, indicating that the 4th layer (HRP-human inhibitor antibody to 80/77 kD is binding to the complex associated to a Class III monoclonal antibody reactive with the 240 kD polypeptide, as well as the 92.5 kD polypeptide and its precursors, and showing an additional reactivity with a 40 kD thrombin digestion product of the Factor VIIIC material - cf. EP-A-91100360.6. When EGTA and EDTA are added to the assay, HRP activity bound in the assay corresponds only to non-specific binding. These results demonstrate that the 92.5 kD protein is bound to the 80/77 kD doublet through calcium bridges.

# EP 0 152 746 B1

## Table

### Role of calcium in the association of 80/77 kD doublet to 92.5 kD protein

| Antibody specificity (2nd layer) | CaCl$_2$ | | CaCl + EGTA + EDTA | |
|---|---|---|---|---|
| | $A_{495}$ sample | $\dfrac{A_{495}\ \text{sample}}{A_{495}\text{NSB*}}$ | $A_{495}$ sample | $\dfrac{A_{495}\ \text{sample}}{A_{495}\text{NSB*}}$ |
| Non–specific binding* | 0.229 | 1 | 0.068 | 1 |
| Medium only** | 0.248 | 1.08 | 0.67 | 0.99 |
| Class III (92.5 kD) | 0.939 | 4.10 | 0.071 | 1.04 |
| Class I (47) (80/77 kD) | 1.81 | 7.9 | 0.523 | 7.69 |

\*  Non–specific binding (NSB) was measured by the ELISA assay deleting the FVIIIC material.

\*\*  Medium only was added instead of the monoclonal antibody (2nd layer) in the ELISA assay.

Additional assays were performed to characterize the monoclonal antibodies in terms of the epitopes they recognized. Competition assays were performed by reacting Factor VIIIC immobilized on microtiter wells with $^{125}$I-42 IgG or $^{125}$I-47 IgG and unlabelled antibodies obtained from cell lines 42, 47, 56 and I-28 at various dilutions. The results showed that monoclonal antibodies obtained from cell lines 47 and 42 react with different epitopes. They also showed that it is likely that monoclonal antibodies obtained from cell line 56 react with an epitope near the binding site of monoclonal antibodies obtained from cell line 42 and induce a conformation change in VIIIC that enhances the affinity for antibodies obtained from cell line 42.

## Claims

1.  The hybridoma cell lines deposited with the National Collection of Animal Cell Cultures, Porton Down, Salisbury, Wilts, GB, under accession numbers 84122101 (cell line 1-28), 84122102 (cell line 47), 84122103 (cell line 42) and 84122104 (cell line 56), and the hybridoma cell line deposited with the American Type Culture Collection, 12301 Parklawn Drive, Rockville, Maryland 20852, US, under accession number HB 8690 (cell line 2B6).

2.  Monoclonal antibodies against human blood coagulation factor VIII, characterized in that they react exclusively with the 80/77 kD doublet fragment or the 80/77 kD doublet fragment and the 240 kD fragment, respectively, as obtainable from any of the hybridoma cell lines of claim 1.

3.  Hybridoma cell lines producing monoclonal antibodies having reactivity identical to that of any of the monoclonal antibodies of claim 2.

4.  Monoclonal antibodies obtained from any of the hybridoma cell lines of claim 3.

## Revendications

1.  Les lignées d'hybridomes déposées auprès de la National Collection of Animal Cell Cultures, Porton Down, Salisbury, Wilts, GB, sous les numéros d'entrée 84122101 (lignée de cellules 1-28), 84122102 (lignée de cellules 47), 84122103 (lignée de cellules 42) et 84122104 (lignée de cellules 56), et la

7

lignée d'hybridomes déposée auprès de l'American Type Culture Collection, 12301 Parklawn Drive, Rockville, Maryland 20852, USA, sous le numéro d'entrée HB 8690 (lignée de cellules 2B6).

2. Anticorps monoclonaux du facteur VIII de la coagulation du sang humain, caractérisés en ce qu'ils réagissent exclusivement avec le fragment de doublet 80/77 kD ou le fragment de doublet 80/77 kD et le fragment 240 kD, respectivement, tels qu'ils peuvent être produits par l'une quelconque des lignées d'hybridomes de la revendication 1.

3. Lignées d'hybridomes produisant des anticorps monoclonaux possédant une réactivité identique à celle de l'un quelconque des anticorps monoclonaux de la revendication 2.

4. Anticorps monoclonaux obtenus par l'une quelconque des lignées d'hybridomes de la revendication 3.

**Patentansprüche**

1. Hybridom-Zellinien, die bei der National Collection of Animal Cell Cultures, Porton Down, Salisbury, Wilts, GB, unter den Hinterlegungsnummern 84122101 (Zellinie 1-28), 84122102 (Zellinie 47), 84122103 (Zellinie 42) und 84122104 (Zellinie 56) hinterlegt sind, sowie die Hybridom-Zellinie, die bei der American Type Culture Collection, 12301 Parklawn Drive, Rockville, Maryland 20852, US, unter der Hinterlegungsnummer HB 8690 (Zelllinie 2B6) hinterlegt ist.

2. Monoklonale Antikörper gegen menschlichen Blutgerinnungsfaktor VIII, dadurch gekennzeichnet, daß sie ausschließlich mit dem 80/77-kD-Dublett-Fragment bzw. dem 80/77-kD-Dublett-Fragment und dem 240-kD-Fragment reagieren, wie aus einer der Zellinien nach Anspruch 1 erhältlich.

3. Hybridom-Zellinien, die monoklonale Antikörper mit einer Reaktivität erzeugen, die gleich der Reaktivität eines der monoklonalen Antikörper nach Anspruch 2 ist.

4. Monoklonale Antikörper, erhalten aus einer der Hybridom-Zellinien nach Anspruch 3.